(19) Europäisches Patentamt
European Patent Office
Office européen des brevets

(11) **EP 1 499 300 B1**

(12) **EUROPEAN PATENT SPECIFICATION**

(45) Date of publication and mention
of the grant of the patent:
**18.03.2009 Bulletin 2009/12**

(51) Int Cl.:
*A61K 9/16* (2006.01)   *A61K 9/22* (2006.01)
*A61K 9/32* (2006.01)   *A61K 9/34* (2006.01)
*A61K 9/36* (2006.01)   *A61K 9/38* (2006.01)
*A61K 9/54* (2006.01)

(21) Application number: **03761018.5**

(22) Date of filing: **29.04.2003**

(86) International application number:
**PCT/US2003/013099**

(87) International publication number:
**WO 2004/000280 (31.12.2003 Gazette 2004/01)**

(54) **PHARMACEUTICAL FORMULATIONS WITH IMPROVED BIOAVAILABILITY**

PHARMAZEUTISCHE FORMULIERUNGEN MIT VERBESSERTER BIOVERFÜGBARKEIT

PREPARATIONS PHARMACEUTIQUES AUX CARACTERISTIQUES BIOLOGIQUES DE
LIBERATION AMELIOREES

(84) Designated Contracting States:
**AT BE BG CH CY CZ DE DK EE ES FI FR GB GR
HU IE IT LI LU MC NL PT RO SE SI SK TR**

(30) Priority: **29.04.2002 US 375803 P**

(43) Date of publication of application:
**26.01.2005 Bulletin 2005/04**

(73) Proprietor: **Supernus Pharmaceuticals, Inc.
Rockville, MD 20850 (US)**

(72) Inventors:
• **SHOJAEI, Amir, H.
Phoenixville, PA 19460 (US)**

• **FLANNER, Henry
Montgomery Village, MD 20886 (US)**
• **IBRAHIM, Scott
Owings Mills, Maryland 21117 (US)**
• **BURNSIDE, Beth, A.
Bethesda, MD 20814 (US)**

(74) Representative: **Walcher, Armin
Louis, Pöhlau, Lohrentz & Segeth
Postfach 3055
90014 Nürnberg (DE)**

(56) References cited:
**US-A- 4 632 843        US-A- 4 940 588
US-A- 5 455 047        US-A- 5 503 852
US-A- 5 958 458**

**Description**

FIELD OF THE INVENTION

The present invention relates to sustained release pharmaceutical formulations with improved absorption and bioavailability of pharmaceutical active agents with limited or region specific absorption and/or pH dependent absorption profile after oral administration.

BACKGROUND OF THE INVENTION

The absorption process of pharmacologically active compounds after oral administration depends, to a large extent, on the physicochemical properties of the active ingredient. This dependency makes certain regions of the intestinal tract more amenable to a given drug's absorption. Optimal absorption is then achieved when the residence time of the drug, at its specific region of absorption, is prolonged. When drug transit through the absorptive region is short, sub-optimal plasma levels are achieved resulting in a short duration of action. Moreover, it makes it difficult to create sustained release formulations for oral administration.

Consequently, the short residence time in the small intestine poses a considerable problem to those skilled in the art interested in developing sustained absorption medicinal products intended for oral administration. The medicinal product administered orally is, in effect, subject to the natural transit of the gastrointestinal tract, thereby limiting its residence time.

US Patent No. 6,267,990 describes controlled release pharmaceutical preparations comprising an ACE inhibitor as active ingredient This patent relates to the use of spherical pellets with two delayed release populations within the delivery system. The system is not retentive, and pH specific coating is the only means by which region specific release is bought about. With such systems, if the pH dependent polymer coating is dissolved too rapidly or too slowly, suboptimal absorption profiles are resulted.

US Patent No. 5,158,777 generally describes a captopril oral delivery system containing spherical beads coated with pH dependent polymer for delayed/enteric release.

US Patents 5,912,013 and 5,326,570, describe dosage forms containing carbamazepine, some of which have been subsequently found to be of irregular shaped particles. However, these particles are not of a sustained release matrix formulation.

US 5,503,852 discloses a microparticulate systel for drug delivery. US 4,632,843 discloses solid pharmaceutical pellets. US 5,958,458 discloses a pharmaceutical multipte unit particulate formulation

It would therefore be advantageous to be able to deliver a drug with a region of absorption limited to the small intestine, particularly the upper half of the small intestine, and increase the drugs residence at this site, which is the preferred location for systemic absorption for a number of active pharmaceutical agents, and be able to make such a dosage form a sustained release matrix formulation.

SUMMARY OF THE INVENTION

The present invention provides a pharmaceutical composition according to claim 1. In accordance with the present invention, there is provided a pharmaceutical formulation for sustained release of an active ingredient in the gastrointestinal tract, comprising a plurality of irregularly shaped cores and wherein the active ingredient is chosen from those agents that have region of absorption limitations, for instance drugs with windows of absorption of less than six hours after ingestion. As examples thereof are certain drugs in the categories of cardiovascular agents, ACE inhibitors, antimicrobials, proton pump inhibitors, antivirals, cancer chemotherapeutic agents, vitamin $B_6$ derivatives, benzodiazepines, analgesics, anticholinergics, anti-ADHD agents, antiepileptics, and phosphodiesterase III inhibitors, although the present invention is not limited to this list.

The present invention provides for such a formulation in the form of a matrix-type sustained release composition. Such a dosage form provides for the sustained release administration of such an active ingredient as mentioned above, which will thereby allow a patient to take fewer dosages during the course of treatment, Ideally, in some situations, the dosage forms of the present invention will allow for a once-a-day dosing regimen.

The present invention also provides a method of making the irregular cores of the present invention, as well as dosage forms containing such cores.

In another aspect, there is provided a a pharmaceutical composition for use in a method for the treatment of a disease comprising administering a formulation in accordance with the present invention to a patient in need of such treatment.

In a further aspect there is provided the use of a formulation in accordance with the present invention for the preparation of a medicament for the treatment of a disease.

Brief description of the drawings

Various other features and attendant advantages of the present invention will be more fully appreciated as the same becomes better understood when considered in conjunction with the accompanying drawings wherein:

Figure 1 shows the dissolution profile of an anagrelide extended-release capsule according to Example 2 (PD0073-124A).

Figure 2 shows the dissolution profile of an anagrelide extended-release capsule according to Example 2 (PD0073-124B).

Figures 3A and B represent photomicrograph of cores made according to the invention (A) compared to sugar spheres (B). Microscope Settings: 25X Lens with 1,000X magnification.

Figure 4 shows the mean dissolution (n=6) profile for trospium chloride from roller compacted granules. Dissolution medium: phosphate buffer, pH 6.8. USP apparatus II, 50 RPM.

DETAILED DESCRIPTION OF THE INVENTION

In one embodiment, compositions and their preparation and use in accordance with the present invention comprise those wherein the following embodiments are present, either independently or in combination.

The current invention provides for irregularly shaped matrix cores containing at least one pharmaceutical active ingredient along with appropriate inactive excipients to yield robust cores. By "irregular" shape is meant, generally, non-spherical.

In order to accommodate for optimal absorption, the prior art has focused on attaining beadlets that are spherical, which will enhance surface area as well as provide particles that are more easily processed, for instance more easily coated. However, such systems fall short of increased retention at the site of absorption due to their geometric configuration. By their nature, sphere-like structures have an increased tendency to "roll down" a given path with the least amount of resistance secondary to friction. Therefore, a non-spherical, irregularly shaped core system was developed which results in increased transit time as compared to a spherical system.

Roundness, or sphericity, is described as the degree of abrasion of a clastic particle as shown by the sharpness of its edges and corners, expressed by Wadell (1932) as the ratio of the average radius of curvature of the several edges or corners of the particle to the radius of curvature of the maximum inscribed sphere (or to one-half the nominal diameter of the particle.) Bates, R. L. and Jackson, J. A., 1980, Glossary of Geology, 2nd Edition. Falls Church, Virginia, American Geological Institute, p. 546. Krumbein et al. describes this visually in Krumbein, W. C. and L. L. Sloss (1951) Stratigraphy and Sedimentation. 2nd. Ed. W. H. Freeman and Company. London.

The majority of the distribution of cores (the production of particles typically results in a bell-shaped distribution of size and shape) of the present invention is not spherical (Wadell sphericity of 1), and preferably has a Wadell sphericity value of 0.7 or less, or a corresponding roundness value of less than 0.40 (subrounded to very angular, see Bates and Jackson, *supra*). More preferably, the majority of the core distribution is between a roundness value of 0.0 and 0.25 (subangular to angular).

Without being bound to any particular theory, it is believed that the cores of the present invention provide for increased retention time based on their geometric configuration; due to their non-spherical and irregular morphologies, they are more apt to get "caught up" in the crevices within the gastrointestinal tract's convoluted morphology of the epithelial barrier.

The increased transit time is also dependent on the force exerted by the gastric or intestinal fluid on the particle as the fluid moves through the GI tract. The force is a result of the relative motion between the cores and the surrounding GI fluid. This can be expressed in mathematical terms in accordance with the following equation.

$$F = CA_p\rho\mu^2/2g_c$$

where C is the coefficient of drag, $A_p$ is the projected particle area in the direction of motion, $\rho$ is the density of the GI fluid, $\mu$ is the relative velocity between the particle and the GI fluid and $g_c$ is the dimensional constant.

The cores of the present invention have a reduced $A_p$ due to their non-spherical, highly irregular shape, thus reducing the value of F leading to slower transit time. The shape factor is a dimensionless number that has been used to mathematically compare the area of an irregularly shaped particle to the area of a sphere of an equivalent volume as the volume of the irregularly shaped particle. The shape factor of a sphere is 1.0 while the shape factor of the irregularly shaped cores is less than 1.0. The more irregularly shaped the particle the lower the shape factor.

In one aspect of the invention, the majority of the irregular shaped cores have a particle size of about 50$\mu$m to about 3000$\mu$m. Preferably, the majority of the irregular shaped cores have a particle size of about 100$\mu$m to about

2000μm. Most preferably, the majority of the irregular shaped cores have a particle size of about 100μm to about 1000μm. The upper limits are desirable to ensure the particles are not too big to hamper further processing, such as filling into capsules. The lower limits are necessary to ensure that the particles contain all of the components required for the matrix character and the drug or drugs. Control of the size of the particles is within the knowledge and skill of one in the art. For instance, with roller compaction, two different screen sizes are utilized to obtain certain size ranges. For example, using mesh 18 and mesh 35 will yield cores of between about 125 and about 800 microns.

The drug (active ingredient) is present within the microparticulates from about 0.1 % (w/w) to 99.0% (w/w), preferably from about 1.0% (w/w) to about 80% (w/w), depending on the drug and dosage.

The pharmaceutically active ingredient is chosen from incompletely absorbed (limited oral bioavailability) pharmaceutical agents, which include *inter alia* certain ACE inhibitors, antimicrobials, benzodiazepines, anticholinergics, muscarinic receptor antagonists, adenosine $A_1$ agonists, and phosphodiesterase inhibitors. The incomplete oral absorption of the active ingredient is due to region specificity for the drug's absorption within the GI tract. The region specificity may be due to the pH of the microenvironment and/or the region's inherent permeability to the active ingredient.

One aspect of the present invention relates to the use of the cores of the present invention for sustained delivery of ACE inhibitors. ACE inhibitors are compounds which inhibit the conversion of angiotensin I to the vasoconstrictor compound angiotensin II as well as the breakdown of the active vasodilator, bradykinin. These activities result in a reduction of peripheral arterial resistance and thus a reduction of blood pressure. ACE inhibitors are being used as effective therapy for hypertension as well as congestive heart failure. Examples of ACE inhibitors that would benefit from being in the dosage forms of the present invention are fasidotril, enalaprilat, or ramipril, or mixtures thereof, which are incompletely absorbed due to the region's inherent permeability to the drug.

Among phosphodiesterase III inhibitors, anagrelide is incompletely absorbed due to a combination of pH and solubility of the active ingredient.

Examples of antimicrobials with region specificity include doxycycline and tetracycline.

An example of an anticholinergic with absorption limitations is trospium chloride. In fact, quaternary ammonium compounds generally have inherently low absorption in the GI tract and as such only certain specific regions (upper duodenum) are optimal for absorption. Thus, other quaternary ammonium compounds would benefit from being composed in accordance with the present invention as well.

Among proton pump inhibitors, all prazole derivatives are region specific due to the pH of the microenvironment. These drugs degrade at low pH (<5).

An example of a muscarinic receptor antagonist is trospium chloride.

Among cancer chemotherapeutic agents are chlorambucil, carboplatin, derivatives of busulfan, doxorubicin, etoposide, and topotecan (TPT), which are incompletely absorbed due to permeability and pH issues.

Among anti-epileptics is particularly gabapentin, which is incompletely absorbed.

Among analgesics that have region specificity are codeine and morphine.

Among benzodiazepines with region specificity are clonazepam, midazolam and triazolam.

Among cardiovascular agents is verapimil.

It will be appreciated by those skilled in the art that the active ingredients can be used in the form of pharmaceutically acceptable salts or esters or derivatives and in the case of chirally active ingredients, one can use both optical isomers, geometric isomers and mixtures thereof, including racemic mixtures. Moreover, the above-listed examples do not comprise a comprehensive list and other drugs with region of absorption concerns are contemplated as useful in the present invention.

The majority (i.e., more than 50%) of the cores of the present invention must have irregular, non-spherical shapes and as such the preparation of the cores is done using technologies capable of such geometric irregularity. One means for the preparation of the cores is through roller compaction of a dry blend. For example, the cores of the present invention can be prepared by screening each component through a mesh sieve and dry blending the mixture in a V-blender. The blended powders are then processed through a roller compactor, where the blends are compacted and dry granulated to form cores of non-spherical shapes. The cores are typically then screened through appropriate meshes such as 18 and 40 mesh sieves.

Another means to obtain cores of the present invention that have irregular and non-spherical/angular morphology is by high shear granulation or roto-granulation (for wet granulation processes). Yet another means for obtaining the cores is by milling (hammer milling, roller milling, etc.) processes that reduce particle size of substrate to the ranges specified herein.

The most preferred approach for manufacture of such cores is the roller compaction technology, through which, unexpectedly, mostly non-spherical cores are obtained according to the present invention.

By "sustained release" is meant a formulation that temporally releases the active ingredient to tissues, or releases drug temporally to be absorbed through the GI tract to the blood stream, thus to the targeted tissue.

The cores are formed into a matrix composition to attain their sustained released nature. Matrix devices can be composed of insoluble plastics, hydrophilic polymers, or hydrophobic or fatty compounds.

Making a matrix composition involves, for example, adding a powdered wax at 5-30% of the total formulation weight, such as hydrogenated castor oil, glyceryl palmitostearate, glyceryl behenate, Gelucire, PEG 8000 or any other known non-swellable matrix forming agent. The wax may be granulated with any component or combination of components of the formulation with a 0-20% PVP K25 or PEG 8000 or other binder solution, and after subjecting to a procedure that favors non-spheroidal particles, the particles are then added to any remainder of the formulation (for tableting or encapsulating, for instance) using known methods.

The cores of the present invention may further be coated with one or more coating agents, such as enteric coatings. Such coating agents are not generally soluble in the stomach environment, are slowly soluble in the GI tract, or are soluble at various pHs.

Hydrophobic or fatty components useful for matrix formulations include, for example, ethyl cellulose, glyceryl monostearate, mixtures of glyceryl monostearate and glyceryl monopalmitate, glyceryl monooleate, a mixture of mono-, di and triglycerides, glyceryl monolaurate, paraffin, white wax, glyceryl dibehenate, long chain carboxylic acids, long chain carboxylic acid esters or long chain carboxylic acid alcohols.

For the preparation of matrix cores one can also include one or more hydrophilic polymers in the formulation. Hydrophilic polymers include, but are not limited to, swellable hydrophilic polymers and non-swellable hydrophilic polymers.

Hydrophilic swellable polymers include, for example, hydroxypropyl methylcellulose, hydroxypropyl cellulose, polymethacrylic acid copolymers, polycarbopols, or polyethylene oxides.

Non-swellable hydrophilic polymers include, for example, polyethylene glycol, ethylcellulose (e.g., Ethocel®), cellulose acetate, cellulose ester butyrate, cellulose acetate proprionate, cellulose acetate phthalate, methacrylic acid and ammoniomethacrylic acid polymers, such as all the Eudragit polymers, Eudragit RS, Eudragit RL, and enteric polymers such as Eudragit L30D-55 and Eudragit FS30D.

Plastics used in matrix tablets include, for instance, methyl acrylate-methyl methacrylate, polyvinyl chloride and polyethylene.

Enteric coating agents include, for instance, polymers that are substantially insoluble in the acidic environment of the stomach, but are predominantly soluble in intestinal fluids at specific pHs. The enteric materials are non-toxic, pharmaceutically acceptable polymers, and include, for example, cellulose acetate phthalate (CAP), hydroxypropyl methylcellulose phthalate (HPMCP), polyvinyl acetate phthalate (PVAP), hydroxypropyl methylcellulose acetate succinate (HPMCAS), cellulose acetate trimellitate, hydroxypropyl methylcellulose succinate, cellulose acetate succinate, cellulose acetate hexahydrophthalate, cellulose propionate phthalate, cellulose acetate maleate, cellulose acetate propionate, copolymer of methylmethacrylic acid and methyl methacrylate, copolymer of methyl acrylate, methylmethacrylate and methacrylic acid, copolymer of methylvinyl ether and maleic anhydride (Gantrez ES series), ethyl methyacrylate-methylmethacrylate-chlorotrimethylammonium ethyl acrylate copolymer, natural resins such as zein, shellac and copal collophorium, and several commercially available enteric dispersion systems (e.g., Eudragit L30D55, Eudragit FS30D, Eudragit L100, Eudragit S100, Kollicoat EMM30D, Estacryl 30D, Coateric, and Aquateric). The foregoing is a list of possible materials, but one of skill in the art would recognize that it is not comprehensive and that there are other enteric materials that would meet the objectives of the present invention of providing for a modified release profile.

Alternatively, the coating can contain one or more polymers that are soluble at various, or different in the case of more than one, pHs. These sustained release coatings will allow for a release delayed until the pH of the environment is such that it will allow the coating to dissolve. Hydroxypropyl methylcellulose phthalate (HPMCP), polyvinyl acetate phthalate (PVAP) and Coateric will dissolve in buffers of pH 5.0 and higher. Eudragit L100-55, Eudragit L30D-55, Kollicoat EMM30D, and Estacryl 30D will dissolve from pH 5.5 to 6.5. Cellulose acetate phthalate (CAP) and Aquateric will dissolve in buffers above pH 6.2. Eudragit S100 and FS30D will dissolve around pH 7.0-7.5. Roughly, the pH of the duodenum is about 5.5, the jejunum is about 6.5 and the distal ileum is about 7.5.

The formulation of the present invention may further include other materials such as permeation/absorption/solubility enhancers or promoters, bulking agents, disintegrating agents, anti-adherants and glidants, lubricants, and binding agents.

Permeation/absorption enhancers or promoters include but are not limited to cationic, anionic, and nonionic surfactants, medium chain glycerides, blends of mono- di-, and triglycerides, or vitamin E TPGS.

Bulking agents include, but are not limited to, microcrystalline cellulose (e.g., Avicel®, FMC Corp., Emcocel®, Mendell Inc.), mannitol, xylitol, dicalcium phosphate (eg. Emcompress, Mendell Inc.) calcium sulfate (eg. Compactrol, Mendell Inc.) starches, lactose, sucrose (Dipac, Amstar, and Nutab, Ingredient Technology), dextrose (Emdex, Mendell, Inc.), sorbitol, cellulose powder (Elcema, Degussa, and Solka Floc, Mendell, Inc.) The bulking agent may be present in the composition in an amount of from about 1 wt. % to about 90 wt. %, preferably from about 10 wt. % to about 50 wt. %.

Disintegrating agents that may be included in the composition include, but are not limited to, microcrystalline cellulose, starches, crospovidone (eg. Polyplasdone XL, International Specialty Products.), sodium starch glycolate (Explotab, Mendell Inc.), and crosscarmellose sodium (eg. Ac-Di-Sol, FMC Corp.). The disintegrating agent may be present in the composition in an amount of from about 0.1 wt. % to about 30 wt %, preferably from about 1 wt. % to about 15 wt. %.

Antiadherants and glidants which may be employed in the composition include, but are not limited to, talc, corn starch, silicon dioxide, sodium lauryl sulfate, and metallic stearates. The antiadherant or glidant may be present in the composition in an amount of from about 0.2 wt. % to about 15 wt. %, preferably from about 0.5 wt. % to about 5 wt. %.

Lubricants which may be employed in the composition include, but are not limited to, magnesium stearate, calcium stearate, sodium stearate, stearic acid, sodium stearyl fumarate, hydrogenated cotton seed oil (sterotex), talc, and waxes, including but not limited to, beeswax, carnuba wax, cetyl alcohol, glyceryl stearate, glyceryl palmitate, glyceryl behenate, hydrogenated vegetable oils, and stearyl alcohol. The lubricant may be present in an amount of from about 0.05 wt. % to about 20 wt. %, preferably from about 0.5 wt. % to about 5 wt. %.

Binding agents which may be employed include, but are not limited to, polyvinyl pyrrollidone, starch, methylcellulose, hydroxypropyl methylcellulose, carboxymethyl cellulose, sucrose solution, dextrose solution, acacia, tragacanth and locust bean gum. The binding agent may be present in the composition in an amount of from about 0.2 wt. % to about 10 wt. %, preferably from about 0.5 wt. % to about 5 wt. %.

A protective coat (e.g., OPADRY® beige or white) also can be applied onto the cores or tablets to provide color or physical protection.

As long as the presentation of the cores, as described in the present invention, in the gastrointestinal tract is in the form of non-spherical irregularly shaped particulates, the dosage form can be prepared as single or multi-layered, coated or uncoated, tablets or beads of a capsule.

The compositions of the present invention may be made into a tablet by any tableting method, such as direct compression, wet or dry granulation, or fluid bed granulation. In the direct compression method, the resultant blends of the cores of the present invention and any other excipients are compressed into tablets on a rotary press using appropriate tooling. In compressing the cores of the invention into a tablet, it is important that the irregularity of the cores be mostly maintained by, for instance using a cushioning type of tablet filler such as microcrystalline cellulose and the like. It will be apparent to one skilled in the art that different agents can be added to the core mixture when preparing the tablet. For example, a disintegrant can be added to the mixture, which will allow the cores to be released in the GI tract. The compressed tablets and/or the cores may be coated, if desired.

For powder forms, such as sachets, no further processing of the cores is necessary. For capsules, which are the preferred dosage forms herein, the cores, which are coated or not, are encapsulated into hard or soft capsules. For any of the dosage forms, there may be mixtures of the cores of the present invention as well as conventional, round cores in order to obtain different, such as pulsatile, release profiles. For example, a tablet or capsule may contain multiple cores or particulates, the concept of which is disclosed, for instance in US Patent No. 6,322,819, which is hereby incorporated herein by reference.

The compositions of the present invention may be employed to treat a variety of diseases or disorders. For example, when the pharmaceutically active agent is anagrelide hydrochloride, the composition may be employed in treating a variety of blood disorders, including, but not limited to, myeloproliferative blood disorders or MBDs, such as, for example, essential thrombocythemia, or ET, chronic myelogenous leukemia, or CML, polycythemia vera, or PV, and agnogenic myeloid metaplasia, or AMM. The composition including anagrelide HCl may be administered to an animal, such as a mammal, including human and non-human primates, in an amount effective to treat such disorders.

Trospium chloride is an antimuscarinic drug used for the treatment of detrusor instability or detrusor hyperreflexia, with the symptoms of urinary frequency, urgency, and incontinence, as well as for the control of spasms in genitourinary tract disorders. It works to prevent smooth muscle contraction such as that found in the bladder by blocking the effects of acetylcholine. Dosage is typically 20 mg twice daily. Fusgen et al., Hauri D. Trospium chloride: an effective option for medical treatment of bladder overactivity. Int J Clin Pharmacal Ther 2000;38:223-234. By use of the present invention, a once a day formulation is attainable. For such a once-a-day dosage, anywhere from about 20 mg. to about 120 mg. of trospium chloride is used, depending on the particular composition of the irregular cores. Preferable is a dosage of about 40 to about 120 mg., and most preferred is a 40 mg. dosage form.

Unless otherwise defined, all technical and scientific terms used herein have the same meaning as commonly understood by one of ordinary skill in the art to which this invention belongs. All publications, patent applications, patents, and other references mentioned herein are incorporated by reference in their entirety. In case of conflict, the present specification, including definitions, will control. In addition, the materials, methods, and examples are illustrative only and not intended to be limiting.

It will be appreciated that the amount of a compound of the invention required for use in treatment will vary not only with the particular compound selected but also with the route of administration, the nature of the condition for which treatment is required and the age and condition of the patient and will be ultimately at the discretion of the attendant physician or veterinarian. In general however a suitable dose will be in the range of from about 0.0001 to about 50 mg/kg of body weight per day, preferably in the range of 0.01 to 40 mg/kg/day, most preferably in the range of 0.015 to 20 mg/kg/day.

The desired dose may conveniently be presented in a single dose or as divided dose administered at appropriate intervals, for example as two, three, four or more doses per day.

The following examples are provided to illustrate various embodiments of the present invention and shall not be considered as limiting in scope.

## Examples

### Example 1

Typical cores compositions are shown in Table 1. Both formulations contain an active pharmaceutical agent. Formulations A and B are prepared by screening each component through a mesh 18 sieve and blending the mixture in a V-blender for 10 minutes. The respective blended powders are then processed through a roller compactor, where the blends are compacted and dry granulated to form cores. The cores are screened through mesh 18 and mesh 40 sieves and the irregular shaped cores in between is collected and encapsulated into size 3 hard gelatin capsules using an encapsulator.

**Table 1. Composition of Core Formulations**

| Ingredients | Formulation A | | Formulation B | |
|---|---|---|---|---|
| | 1 | 2 | 1 | 2 |
| Active Pharmaceutical Agent | 10 | 12 | 10 | 10 |
| Ludipress | 32 | 38 | 30 | 30 |
| Prosolv HD90 | 28 | 34 | 14 | 14 |
| Methocel E5 | 12.50 | 15 | 10 | 10 |
| Methocel K4M | ---- | ---- | 35 | 35 |
| Pruv | 0.83 | 1 | 1 | 1 |
| 1 = composition in mg per capsule<br>2 = composition in % weight<br>Note:<br>Ludipress is a trade name for a blend of lactose, polyvinyl pyrrolidone, and crosslinked polyvinyl pyrrolidone and marketed by BASF Corporation. Prosolv HD90 is a trade name for silicified microcrystalline cellulose and marketed by Penwest Corp.<br>Methocel E5 is a trade name for hydroxypropyl methylcellulose and marketed by the Dow Chemical Company.<br>Methocel K4M is a trade name for hydroxypropyl methylcellulose and marketed by Dow Chemical Company.<br>Pruv is a trade name for sodium stearyl fumarate and is marketed by Penwest Corp. | | | | |

### Example 2

Anagrelide compositions are shown in Table 2. Both formulations contain anagrelide HCl as the active pharmaceutical agent. Formulations PD0073-124A and PD0073-124B were prepared by screening each component through a mesh 18 sieve and blending the mixture in a V-blender for 10 minutes. The respective blended powders were then processed through a roller compactor, where the blends were compacted and dry granulated to form irregular cores. The cores were screened through mesh 18 and mesh 40 sieves and the material in between was collected and encapsulated into size 3 hard gelatin capsules using an encapsulator.

**Table 2. Composition of Core Formulations of Anagrelide HCl**

| Ingredients | PD0073-124A | | PD0073-124B | |
|---|---|---|---|---|
| | 1 | 2 | 1 | 2 |
| Anagrelide HCl | 1.22 | 0.61 | 1.22 | 0.61 |

(continued)

|  | PD0073-124A | | PD0073-124B | |
| Ingredients | 1 | 2 | 1 | 2 |
| --- | --- | --- | --- | --- |
| Polyox WSR 301 | 80 | 40 | --- | --- |
| Avicel pH101 | 30 | 15 | 10 | 5 |
| Fujicalin | 30 | 15 | --- | --- |
| Eudrgait S100 | 30 | 15 | 30 | 15 |
| Eudragit L100 | --- | --- | 20 | 10 |
| Fumaric acid | 10 | 5 | --- | --- |
| Ethocel | --- | --- | 80 | 40 |
| Eudragit RS | --- | --- | 20 | 10 |
| Compritol | 18.78 | 9.39 | 38.78 | 19.39 |

1 = composition in mg per capsule
2 = composition in % weight
Note: Polyox WSR 301 is a trade name for poly(ethylene oxide) and is marketed by Union Carbide. Avicel pH 101 is a trade name for microcrystalline cellulose and is marketed by FMC Biopolymer. Fujicalin is a trade name for dibasic calcium phosphate and is marketed by Fuji Chemical Industry Co., Ltd. Eudragit S100 is a trade name for is a trade name for poly(methacrylic acid-co-ethyl acrylate) and is marketed by Rohm GmbH. Eudragit L100 is a trade name for poly(methacrylic acid-co-ethyl acrylate) and is marketed by Rohm GmbH. Ethocel is a trade name for ethylcellulose and is marketed by the Dow Chemical Company. Eudragit RS is a trade name for poly (methacrylic acid-co-ethyl acrylate) and is marketed by Rohm GmbH. Compritol is a trade name for glyceryl behenate and is marketed by Gattefosse.

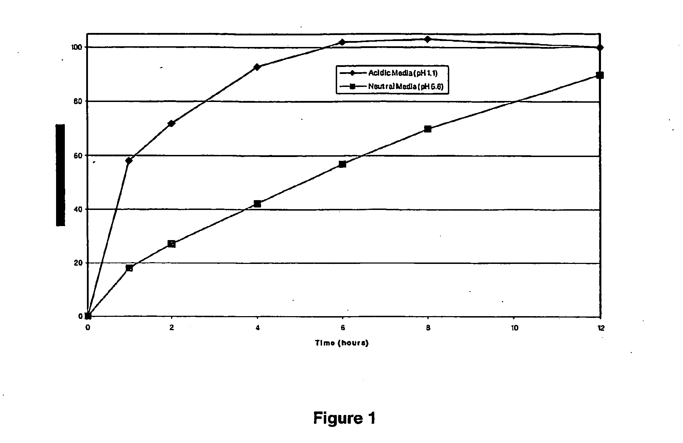

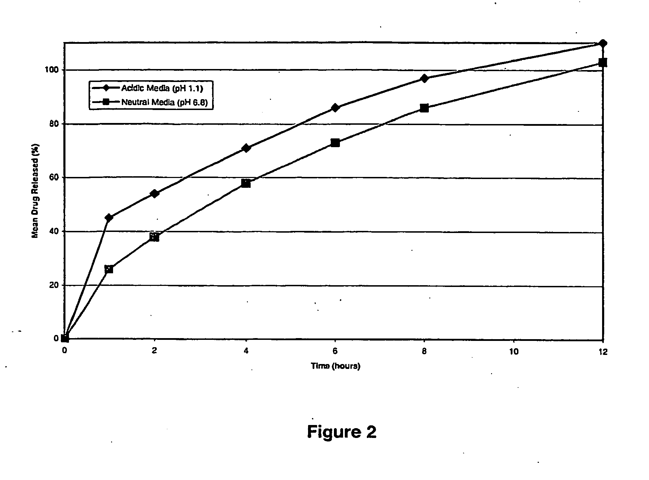

Anagrelide irregular cores formulations were tested for drug release as a function of time in a USP dissolution apparatus using acid media as well as neutral (pH 6.8) media. The results are shown in Figures 1 and 2. Both formulations were able to sustain the release of anagrelide over a prolonged period of time.

### Example 3

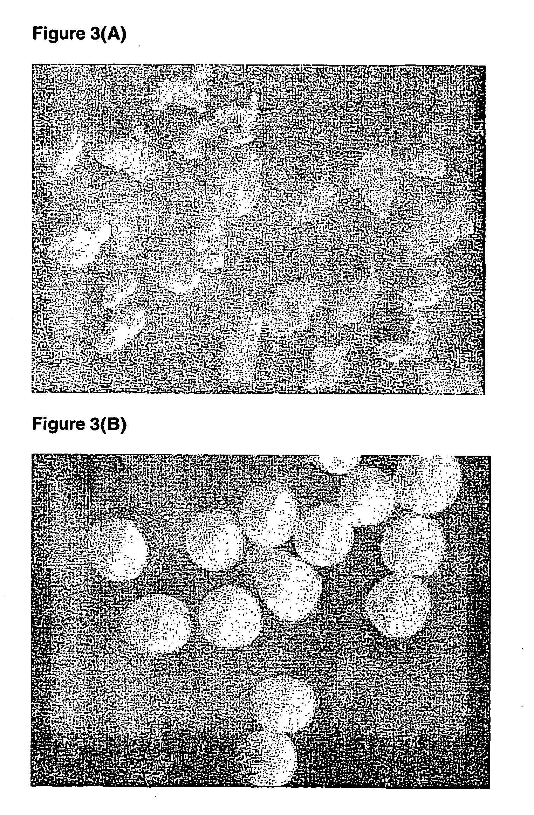

To depict the irregular and non-spherical morphology of the cores, the subject of this invention, cores were developed and photographed. The cores were developed according to the procedure outlined in Example 1. Figure 3A shows cores in accordance to the current invention and Figure 3B shows 25-35 mesh sugar spheres. The cores of Figure 3A can be rated as 0.1 to 0.2 on the Krumbein visual scale.

**Example 4 -** Roller compacted trospium preparation for extended release

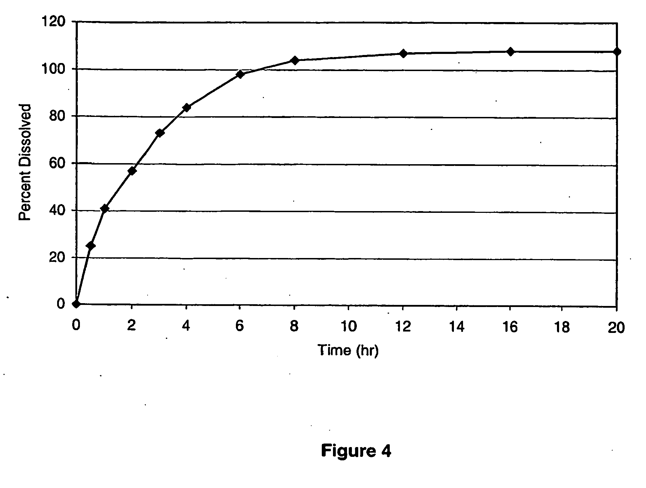

The composition is shown in Table 3. Drug and excipients were screened through an 18-mesh sieve and mixed in a V-blender for 5 minutes. The blend was then roller compacted. The roller compactor processing parameters are: roller speed = 8 rpm; feed screw speed = 25 rpm; granulator speed = 80 rpm; roller pressure = 100 bar; and top/bottom screens are 1.25mm/0.63mm. Granules obtained were screened and those retained between 20- and 50-mesh sieves were collected. Granules were encapsulated in size 0 white opaque coni-snap capsules and analysed for dissolution. Figure 4 shows the mean dissolution profile for PD0150-182E.

**Table 3**

| Ingredients | % Composition |
| --- | --- |
| Trospium Chloride | 20 |

(continued)

| Ingredients | % Composition |
|---|---|
| Prosolv HD90 | 40 |
| Compritol 888ATO | 20 |
| Klucel EXF | 10 |
| Kollidon K30 | 10 |
| Note: Klucel EXF is hydroxypropylcellulose, available from Hercules, Inc., Delaware. Kollidon K30 is povidone, manufactured by BASF. | |

### Example 5

The following formulations were blended as described in the previous examples, and roller compacted with the following parameters: top screen: 1.25 mm, bottom screen: 0.8 mm; feed screw speed =19 rpm; roller speed = 8 rpm; granulator speed = 70 rpm; and roller pressure = 150 bar.

| Ingredient | %w/w |
|---|---|
| Topiramate | 40 |
| Avicel 301 | 39 |
| HPMC K15 CR | 20 |
| Magnesium Stearate | 1 |

| Ingredient | %w/w |
|---|---|
| Topiramate | 40 |
| Compritol 888ATO | 30 |
| Prosolv HD90 | 24 |
| HPMC K15 CR | 5 |

### Example 6

The following formulations were blended as described in the previous examples, and roller compacted with the following parameters: top screen: 1.25 mm, bottom screen: 0.63 mm; feed screw speed = 25 rpm; roller speed = 8 rpm; granulator speed = 80 rpm; and roller pressure = 100 bar.

| Ingredient | %w/w |
|---|---|
| Trospium Chloride | 20 |
| Klucel EF | 10 |
| Prosolv HD90 | 20 |
| Kollidon K30 | 10 |
| Compritol ATO888 | 20 |
| Methocel K100M | 20 |

| Ingredient | %w/w |
|---|---|
| Trospium Chloride | 20 |
| Klucel EXF | 10 |
| Prosolv HD90 | 40 |
| Kollidon K30 | 10 |
| Compritol ATO888 | 20 |

## Example 7

SLI460 is an adenosine $A_1$ agonist. All ingredients in each of the tables below were sieved through a size 30 mesh screen. Formulation was PK blended without API (active pharmaceutical ingredient) for 5 minutes without intensifier bar. Upon adding API to blend, formulation was blended 3 minutes with intensifier bar, then additional 2 minutes without intensifier bar. Formulation was then passed through the roller compactor, collecting microparticulates between sizes 18-35 mesh sieves. The remaining particles were passed through the roller compactor and collected in the same manner for an additional 2 passes.

Blends were roller compacted with the following parameters: top screen: 2.5 mm, bottom screen: 1.0 mm; feed screw speed = 25 rpm; roller speed = 8 rpm; granulator speed = 70 rpm; and roller pressure = 100 bar.

| Ingredient | %w/w |
|---|---|
| SLI460 | 1.5 |
| Fumaric Acid | 5 |
| Methocel K15 CR | 13.5 |
| Compritol 888ATO | 15 |
| Ludipress | 40 |
| Polyox WSR | 15 |
| Avicel 101 | 10 |

| Ingredient | %w/w |
|---|---|
| SLI460 | 1.5 |
| Fumaric Acid | 5 |
| Methocel K15 CR | 29 |
| Compritol 888ATO | 15 |
| Ludipress | 18.5 |
| Polyox WSR | 30 |
| Avicel 101 | 1 |

| Ingredient | %w/w |
|---|---|
| SL1460 | 1.5 |
| Fumaric Acid | 0.5 |
| Methocel K15 CR | 30 |
| Compritol 888ATO | 25 |

(continued)

| Ingredient | %w/w |
| --- | --- |
| Eudragit RSPO | 41.5 |
| Avicel 101 | 1.5 |

The preceding examples can be repeated with similar success by substituting the generically or specifically described reactants and/or operating conditions of this invention. From the foregoing description, one skilled in the art can easily ascertain the essential characteristics of this invention and, without departing from the spirit and scope thereof, can make various changes and modifications of the invention to adapt it to various usages and conditions.

**Claims**

1. A pharmaceutical composition of an active pharmaceutical agent with a limited oral bioavailability comprising a plurality of cores containing said active agent in a matrix formulation, wherein the majority of the cores are substantially non-spherical cores with a sphericity of less than 0.7, and wherein the sphericity is the ratio of an average radius of curvature of the several edges or corners of a particle to the radius of curvature of the maximum inscribed sphere.

2. The composition of claim 1, wherein the cores are formed into a tablet while maintaining their substantially non-spherical shape.

3. The composition of claim 2, wherein the tablet is coated with at least one enteric or sustained release coating.

4. The composition of claim 1, wherein the cores are filled into a capsule.

5. The composition of claim 4, wherein the capsule contains coated and uncoated cores.

6. The composition of claim 1, wherein the active agent is selected from ACE inhibitors, antimicrobials, benzodiazepines, anticholinergics, muscarinic receptor antagonists, adenosine A.sub.1 agonists, and phosphodiesterase inhibitors.

7. The composition of claim 1, wherein the active agent is a quaternary ammonium compound.

8. The composition of claim 6, wherein the active agent is trospium or a salt thereof.

9. The composition of claim 8, wherein the active agent is trospium chloride.

10. The composition of claim 6, wherein the active agent is an adenosine A.sub.1 agonist.

11. The composition of claim 3, wherein said enteric coating is one or more selected from cellulose acetate phthalate (CAP), hydroxypropyl methylcellulose phthalate (HPMCP), polyvinyl acetate phthalate (PVAP), hydroxypropyl methylcellulose acetate succinate (HPMCAS), cellulose acetate trimellitate, hydroxypropyl methylcellulose succinate, cellulose acetate succinate, cellulose acetate hexahydrophthalate, cellulose propionate phthalate, cellulose acetate maleate, cellulose acetate propionate, copolymer of methylmethacrylic acid and methyl methacrylate, copolymer of methyl acrylate, methylmethacrylate and methacrylic acid, copolymer of methylvinyl ether and maleic anhydride (Gantrez ES series), ethyl methyacrylate-methylmethacrylate-chlorotrimethylammonium ethyl acrylate copolymer, zein, shellac, copal colophonium, Eudragit L30D55, Eudragit FS30D, Eudragit L100, Eudragit S100. Kollicoat EMM30D, Estacryl 30D, Coateric, and Aquateric.

12. The composition of claim 3, wherein said sustained release coating is one or more selected from hydroxypropyl methylcellulose phthalate (HPMCP), Polyvinyl acetate phthalate (PVAP), Coateric, Eudragit L100-55, Eudragit L30D-55, Kollicoae EMM30D, Estacryl 30D, cellulose acetate phthalate (CAP), Aquateric, Eudragit S100, and Eudragit FS30D.

13. A process of preparing cores of pharmaceutically active agents, comprising mixing a pharmaceutically agent that has a limited oral bioavailibility with one or more matrix materials to thereby form a matrix core, and subjecting the resulting mixture to one of roller compaction, hammer milling or roller milling.

14. The process of claim 13, further comprising the step of compressing the cores produced thereby into a tablet.

15. The process of claim 14, further comprising coating said tablet with one or more enteric coatings.

16. The process of claim 13, wherein said matrix materials are selected from insoluble plastics, hydrophilic polymers or hydrophobic/fatty compounds.

17. The process of claim 16, wherein said hydrohdbic/fatty compounds are one or more of ethyl cellulose, glyceryl monostearate, mixtures of glyceryl monostearate and glyceryl monopalmitate, glyceryl monooleate, a mixture of mono-, di and triglycerides, glyceryl monolaurate, paraffin, white wax, glyceryl dibehenate, long chain carboxylic acids, long chain carboxylic acid esters and/or long chain carboxylic acid alcohols.

18. The process of claim 17, wherein said hydrophobic/fatty compounds are selected from hydrogenated castor oil, glyceryl palmitostearate, glyceryl behenate, glyceryl esters of C12-18 fatty acids, and/or polyethylene glycol.

19. The process of claim 16, wherein said hydrophilic polymers are selected from hydroxypropyl methylcellulose, hydroxypropyl cellulose, polymethacrylic acid copolymers, polycarbopols, polyethylene oxides, polyethylene glycol, ethylcellulos cellulose acetate, cellulose ester butyrate, cellulose acetate propionate cellulose acetate phthalate, and methacrylic acid methylmethacrylate copolymers.

20. The process of claim 19, wherein said hydrophilic polymer is hydroxypropyl methylcellulose.

21. The process of claim 13, wherein said insoluble plastic is selected from methyl acrytate-methyl methacrylate, polyvinyl chloride and polyethylene

22. A pharmaceutical composition according to any one of claims 1 to 12 for use in a method of treating a patient with a sustained release pharmaceutically active agent.

23. The composition of claim 2, additionally comprising a cushioning type tablet filler to maintain the substantially non-spherical shape of the cores.


**Patentansprüche**

1. Pharmazeutische Zusammensetzung eines pharmazeutischen Wirkstoffes mit begrenzter oraler Bioverfügbarkeit, umfassend eine Mehrzahl von Kernen, die den Wirkstoff in einer Matrixzubereitung enthalten, wobei es sich beim Großteil der Kerne um im Wesentlichen nicht-sphärische Kerne mit einer Sphärizität von weniger als 0,7 handelt und wobei es sich bei der Sphärizität um das Verhältnis eines durchschnittlichen Krümmungsradius der mehreren Kanten oder Ecken eines Teilchens zum Krümmungsradius der maximalen eingeschriebenen Kugel handelt.

2. Zusammensetzung nach Anspruch 1, wobei die Kerne zu einer Tablette geformt sind, während ihre im Wesentlichen nicht-sphärische Gestalt aufrechterhalten bleibt.

3. Zusammensetzung nach Anspruch 2, wobei die Tablette mit mindestens einem enterischen Überzug oder einem Überzug mit verzögerter Freisetzung beschichtet ist.

4. Zusammensetzung nach Anspruch 1, wobei die Kerne in eine Kapsel gefüllt sind.

5. Zusammensetzung nach Anspruch 4, wobei die Kapsel beschichtete und unbeschichtete Kerne enthält.

6. Zusammensetzung nach Anspruch 1, wobei der Wirkstoff aus ACE-Inhibitoren, antimikrobiellen Mitteln, Benzodiazepinen, Anticholinergika, Muscarin-Rezeptor-Antagonisten, Adenosin A.sub.1-Agonisten und Phosphodiesterase-Inhibitoren ausgewählt ist.

7. Zusammensetzung nach Anspruch 1, wobei es sich beim Wirkstoff um eine quaternäre Ammoniumverbindung handelt.

8. Zusammensetzung nach Anspruch 6, wobei es sich beim Wirkstoff um Trospium oder ein Salz davon handelt.

9. Zusammensetzung nach Anspruch 8, wobei es sich beim Wirkstoff um Trospium-chlorid handelt.

10. Zusammensetzung nach Anspruch 6, wobei es sich beim Wirkstoff um einen Adenosin A.sub.1-Agonisten handelt.

11. Zusammensetzung nach Anspruch 3, wobei es sich beim enterischen Überzug um einen oder mehrere Bestandteile handelt, die ausgewählt sind aus: Celluloseacetatphthalat (CAP), Hydroxypropylmethylcellulosephthalat (HPMCP), Polyvinylacetatphthalat (PVAP), Hydroxypropylmethylcelluloseacetatsuccinat (HPMCAS), Celluloseacetattrimellitat, Hydroxypropylmethylcellulosesuccinat, Celluloseacetatsuccinat, Celluloseacetathexahydrophthalat, Cellulosepropionatphthalat, Celluloseacetatmaleat, Celluloseacetatpropionat, Copolymeres von Methylmethacrylsäure und Methylmethacrylat, Copolymeres von Methylacrylat, Methylmethacrylat und Methacrylsäure, Copolymeres von Methylvinylether und Maleinsäureanhydrid (Gantrez-ES-Serie), Ethylmethyacrylat-Methylmethacrylat-Chlortrimethylammoniumethylacrylat-Copolymeres, Zein, Schellack, Copal-Kolophonium, Eudragit L30D55, Eudragit FS30D, Eudragit L100, Eudragit S100, Kollicoat EMM30D, Estacryl 30D, Coateric und Aquateric.

12. Zusammensetzung nach Anspruch 3, wobei es sich beim Überzug mit verzögerter Freisetzung um einen oder mehrere der Bestandteile handelt, die ausgewählt sind aus: Hydroxypropylmethylcellulosephthalat (HPMCP), Polyvinylacetatphthalat (PVAP), Coateric, Eudragit L100-55, Eudragit L30D-55, Kollicoat EMM30D, Estacryl 30D, Celluloseacetatphthalat (CAP), Aquateric, Eudragit S100 und Eudragit FS30D.

13. Verfahren zur Herstellung von Kernen von pharmazeutischen Wirkstoffen, umfassend das Vermischen eines pharmazeutischen Mittels, das eine begrenzte orale Bioverfügbarkeit aufweist, mit einem oder mehreren Matrixmaterialien unter Bildung eines Matrixkerns und Behandeln des erhaltenen Gemisches durch Walzenverdichten, Mahlen mit einer Hammermühle oder Mahlen mit einem Walzwerk.

14. Verfahren nach Anspruch 13, ferner umfassend die Stufe des Verdichtens der gebildeten Kerne zu einer Tablette.

15. Verfahren nach Anspruch 14, ferner umfassend das Beschichten der Tablette mit einem oder mehreren enterischen Überzügen.

16. Verfahren nach Anspruch 13, wobei die Matrixmaterialien aus unlöslichen Kunststoffen, hydrophilen Polymeren oder hydrophoben/fettartigen Verbindungen ausgewählt sind.

17. Verfahren nach Anspruch 16, wobei es sich bei den hydrophoben/fettartigen Verbindungen um einen oder mehrere der folgenden Bestandteile handelt: Ethylcellulose, Glycerylmonostearat, Gemische von Glycerylmonostearat und Glycerylmonopalmitat, Glycerylmonooleat, ein Gemisch aus Mono-, Di- und Triglyceriden, Glycerylmonolaurat, Paraffin, weißes Wachs, Glyceryldibehenat, langkettige Carbonsäuren, langkettige Carbonsäureester und/oder langkettige Carbonsäurealkohole.

18. Verfahren nach Anspruch 17, wobei die hydrophoben/fettartigen Verbindungen ausgewählt sind aus hydriertem Rizinusöl, Glycerylpalmitostearat, Glycerylbehenat, Glycerylester von C12-18-Fettsäuren und/oder Polyethylenglykol.

19. Verfahren nach Anspruch 16, wobei die hydrophilen Polymeren ausgewählt sind aus Hydroxypropylmethylcellulose, Hydroxypropylcellulose, Polymethacrylsäure-Copolymeren, Polycarbopolen, Polyethylenoxiden, Polyethylenglykol, Ethylcellulose, Celluloseacetat, Celluloseesterbutyrat, Celluloseacetatpropionat, Celluloseacetatphthalat und Methacrylsäure-Methylmethacrylat-Copolymeren.

20. Verfahren nach Anspruch 19, wobei es sich beim hydrophilen Polymeren um Hydroxypropylmethylcellulose handelt.

21. Verfahren nach Anspruch 13, wobei der unlösliche Kunststoff aus Methylacrylat-Methylmethacrylat, Polyvinylchlorid und Polyethylen ausgewählt ist.

22. Pharmazeutische Zusammensetzung nach einem der Ansprüche 1 bis 12 zur Verwendung in einem Verfahren zur Behandlung eines Patienten mit einem verzögert freigesetzten pharmazeutischen Wirkstoff.

23. Zusammensetzung nach Anspruch 2, zusätzlich umfassend einen Tablettenfüllstoff vom Dämpfungstyp, um die im Wesentlichen nicht-sphärische Gestalt der Kerne aufrechtzuerhalten.

**Revendications**

1. Composition pharmaceutique d'un agent pharmaceutique actif ayant une biodisponibilité orale limitée comprenant une pluralité de noyaux contenant ledit agent actif dans une formulation de matrice, dans laquelle les noyaux pour la majeure partie sont des noyaux sensiblement non sphériques avec une sphéricité inférieure à 0,7, et dans laquelle la sphéricité est le rapport entre un rayon de courbure moyen de la pluralité de bords ou coins d'une particule et le rayon de courbure de la sphère inscrite maximale.

2. Composition selon la revendication 1, dans laquelle les noyaux sont formés en un comprimé tout en maintenant leur forme sensiblement non sphérique.

3. Composition selon la revendication 2, dans laquelle le comprimé est enrobé d'au moins un enrobage à libération prolongée ou entérique.

4. Composition selon la revendication 1, dans laquelle les noyaux remplissent une capsule.

5. Composition selon la revendication 4, dans laquelle la capsule contient des noyaux enrobés et non enrobés.

6. Composition selon la revendication 1, dans laquelle l'agent actif est choisi parmi les inhibiteurs d'ACE, les antimicrobiens, les benzodiazépines, les agents anticholinergiques, les antagonistes de récepteur muscarinique, les agonistes de l'adénosine A.sub.1 et les inhibiteurs de phosphodiestérase.

7. Composition selon la revendication 1, dans laquelle l'agent actif est un composé ammonium quaternaire.

8. Composition selon la revendication 6, dans laquelle l'agent actif est le trospium ou un sel de celui-ci.

9. Composition selon la revendication 8, dans laquelle l'agent actif est le chlorure de trospium.

10. Composition selon la revendication 6, dans laquelle l'agent actif est un agoniste d'adénosine A.sub.1.

11. Composition selon la revendication 3, dans laquelle ledit enrobage entérique est constitué d'un ou plusieurs éléments choisis parmi l'acétate phtalate de cellulose (CAP), le phtalate d'hydroxypropyl méthylcellulose (HPMCP), le poly (acétate phtalate de vinyle) (PVAP), l'acétate succinate d'hydroxypropyl méthylcellulose (HPMCAS), l'acétate trimellitate de cellulose, le succinate d'hydroxypropyl méthylcellulose, l'acétate succinate de cellulose, l'acétate hexahydrophtalate de cellulose, le propionate phtalate de cellulose, l'acétate maléate de cellulose, l'acétate propionate de cellulose, un copolymère d'acide méthylméthacrylique et de méthacrylate de méthyle, un copolymère d'acrylate de méthyle, de méthacrylate de méthyle et d'acide méthacrylique, un copolymère de méthylvinyl éther et d'anhydride maléique (gamme Gantrez ES), un copolymère de méthacrylate d'éthyle-méthacrylate de méthyle-éthyl acrylate de chlorotriméthylammonium, la zéine, la gomme laque, le copal colophane, Eudragit L30D55, Eudragit FS30D, Eudragit L100, Eudragit S100, Kollicoat EMM30D, Estacryl 30D, Coateric et Aquateric.

12. Composition selon la revendication 3, dans laquelle ledit enrobage à libération prolongée est constitué d'un ou plusieurs éléments choisis parmi le phtalate d'hydroxypropyl méthylcellulose (HPMCP), le poly(acétate phtalate de vinyle) (PVAP), Coateric, Eudragit L100-55, Eudragit L30D-55, Kollicoat EMM30D, Estacryl 30D, l'acétate phtalate de cellulose (CAP), Aquateric, Eudragit S100 et Eudragit FS30D.

13. Procédé de préparation de noyaux d'agents pharmaceutiquement actifs, comprenant le mélange d'un agent pharmaceutique qui a une biodisponibilité orale limitée avec un ou plusieurs matériaux de matrice pour former ainsi un noyau de matrice, et la soumission du mélange résultant à l'un d'un compactage au rouleau, d'un broyage au marteau ou d'un broyage au rouleau.

14. Procédé selon la revendication 13, comprenant en outre l'étape de compression des noyaux, les transformant ainsi en comprimé.

15. Procédé selon la revendication 14, comprenant en outre l'enrobage dudit comprimé avec un ou plusieurs enrobages entériques.

16. Procédé selon la revendication 13, dans lequel lesdits matériaux de matrice sont choisis parmi les matières plastiques insolubles, les polymères hydrophiles ou les composés hydrophobes/gras.

17. Procédé selon la revendication 16, dans lequel lesdits composés hydrophobes/gras sont constitués d'un ou plusieurs éléments parmi l'éthyl cellulose, le monostéarate de glycéryle, les mélanges de monostéarate de glycéryle et de monopalmitate de glycéryle, le monooléate de glycéryle, un mélange de mono-, di- et triglycérides, le monolaurate de glycéryle, la paraffine, la cire blanche, le dibéhénate de glycéryle, les acides carboxyliques à longue chaîne, les esters d'acides carboxyliques à longue chaîne et/ou les alcools d'acides carboxyliques à longue chaîne.

18. Procédé selon la revendication 17, dans lequel lesdits composés hydrophobes/gras sont choisis parmi l'huile de ricin hydrogénée, le palmitostéarate de glycéryle, le béhénate de glycéryle, les esters de glycéryle d'acides gras en $C_{12}$ à $C_{18}$, et/ou le poly(éthylène glycol).

19. Procédé selon la revendication 16, dans lequel lesdits polymères hydrophiles sont choisis parmi l'hydroxypropyl méthylcellulose, l'hydroxypropyl cellulose, les copolymères de poly(acide méthacrylique), les poly(carbopols), les poly(oxydes d'éthylène), le poly(éthylène glycol), l'éthylcellulose, l'acétate de cellulose, l'ester butyrate de cellulose, l'acétate propionate de cellulose, l'acétate phtalate de cellulose et les copolymères d'acide méthacrylique et de méthacrylate de méthyle.

20. Procédé selon la revendication 19, dans lequel ledit polymère hydrophile est l'hydroxypropyl méthylcellulose.

21. Procédé selon la revendication 13, dans lequel ladite matière plastique insoluble est choisie parmi l'acrylate de méthyle-méthacrylate de méthyle, le poly(chlorure de vinyle) et le poly(éthylène).

22. Composition pharmaceutique selon l'une quelconque des revendications 1 à 12, pour une utilisation dans un procédé de traitement d'un patient avec un agent pharmaceutiquement actif à libération prolongée.

23. Composition selon la revendication 2, comprenant également une charge de comprimé de type amortissement pour maintenir la forme sensiblement non sphérique des noyaux.

**Figure 1**

**Figure 2**

**Figure 3(A)**

**Figure 3(B)**

**Figure 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6267990 B
- US 5158777 A
- US 5912013 A
- US 5326570 A
- US 5503852 A
- US 4632843 A
- US 5958458 A
- US 6322819 B

### Non-patent literature cited in the description

- **BATES, R. L. ; JACKSON, J. A.** Glossary of Geology. American Geological Institute, 1980, 546
- **KRUMBEIN et al.** Stratigraphy and Sedimentation. W. H. Freeman and Company, 1951
- **FUSGEN et al.** Hauri D. Trospium chloride: an effective option for medical treatment of bladder overactivity. *Int J Clin Pharmacal Ther,* 2000, vol. 38, 223-234